# EUROPÄISCHE PATENTSCHRIFT

(11) **EP 0 842 421 B1**
(45) Veröffentlichungstag und Bekanntmachung des Hinweises auf die Patenterteilung: **12.10.2005**
(21) Anmeldenummer: 96902928.9
(22) Anmeldetag: 26.01.1996
(51) Int. Cl.: G01N 33/487, G01N 33/49, G01N 27/48

(54) **MOBILES HANDGERÄT MIT BIOSENSOR**
PORTABLE HAND-HELD DEVICE WITH A BIOSENSOR
APPAREIL PORTABLE DOTE D'UN BIOCAPTEUR

(30) Priorität: 10.07.1995 DE 29511566 U
(43) Veröffentlichungstag der Anmeldung: 20.05.1998
(73) Patentinhaber: ABT Advanced Bioanalytical Technology GmbH, 01454 Radeberg (DE)
(72) Erfinder: KLIMES, Norbert, D-13187 Berlin (DE); PFEIFFER, Dorothea, D-13156 Berlin (DE); SZEPONIK, Jan, D-13187 Berlin (DE); NENTWIG, Jürgen, D-12619 Berlin (DE); SCHELLER, Frieder, D-16341 Zepernick (DE)
(74) Vertreter: Baumbach, Friedrich
(86) Internationale Anmeldenummer: PCT/EP1996/000328
(87) Internationale Veröffentlichungsnummer: WO 1997/003355

(56) Entgegenhaltungen:
- EP-A- 0 520 443
- DE-A- 4 115 792
- FR-A- 2 535 964

## Beschreibung

Die Erfindung betrifft ein Handgerät zur Untersuchung von biologischen Flüssigkeiten wie Vollblut, Liquor, Urin und Serum ohne aufwendige Präanalytik mit Hilfe von elektrochemischen Biosensoren.

Biosensoren werden seit geraumer Zeit als empfindliche und selektive Detektionsmethoden in der Analytik, insbesondere der medizinischen Diagnostik verwendet. Dazu werden die entsprechenden Biosensoren in Analysatoren eingebracht, die in zentralen Laboratorien mit hohem Probenanfall eine schnelle, preiswerte und vor allem richtige Untersuchung der entsprechenden Parameter zulassen. Ein Probendurchsatz von 80 - 180/h ist dazu in der Regel notwendig.
Die Analysatoren basieren auf unterschiedlichen Prinzipien. Größtenteils arbeiten sie mit einem kontinuierlichen, luftsegmentierten Fluß der entsprechenden hochverdünnten Laborproben, das heißt die Methode bedarf mit der notwendigen Verdünnung des Analysenmaterials einer Vorbereitung der zu untersuchenden Lösungen (z.B. Vollblut). Einzelproben sind auf diesen Aparaturen sehr aufwendig zu analysieren, da die Effektivität der Analysatoren erst nach der Inbetriebnahme bei Abarbeiten hoher Probenzahlen zum Tragen kommt (ESAT® 6660 und ECA 180 der Firma Prüfgeräte-Werk Medingen GmbH, EBIO (eppendorf®) der Eppendorf-Netheler-Hinz GmbH).
Weiterhin sind Anordnungen bekannt, die Einzelproben, auch ohne Präanalytik, messen können, die allerdings auf Grund von hohem integrierten Meßkomfort sehr unbeweglich und damit für den dezentralen Einsatz unbrauchbar sind (YSI 2300 STAT der YSI Incorporated sowie STAT-Profile PLUS der NOVA BIOMEDICAL GmbH).
Aus EP-A-0 520 443 ist eine tragbare Sensorvorrichtung bekannt, die aus Abfallkollektor, Flüssigkeitskanälen und - einlässen sowie elektrochemischem Sensor in einem Gehäuse und externer Kalibriereinheit besteht.

Gegenwärtig kommerzialisierte mobile Systeme sind mit integrierten Biosensoren auf Grund von hohem Meßkomfort (interne Kalibrierung) zu teuer, haben zu hohen Wartungsaufwand und arbeiten mit zu großen Transportwegen für die zu messende Probe. Daraus ergeben sich neben einem relativ hohen Preis Verzögerungen und Verunreinigungen, die sich nachteilig auf die analytische Qualität auswirken.

Die im dezentralen Einsatz befindlichen Meßsysteme zur Bestimmung von Parametern wie Glucose und Lactat basieren momentan alle auf nicht wiederverwendbaren Verbrauchsmaterialien (Teststreifen, photometrisch; Streifenelektrode, amperometrisch). Damit ist die Analyse durch den Preis des Teststreifens determiniert und der Teststreifen ist nicht kalibrierbar, da nur einmal verwendbar.

Aufgabe der Erfindung war es deshalb, die technische Lösung für die Realisierung eines dezentral einsetzbaren Gerätes zur Untersuchung von Blut, Urin, Liquor ohne Präanalytik und wiederverwendbarem Biosensor zu schaffen, das sich durch einen niedrigen Preis als auch niedrigstem Wartungsaufwand auszeichnet.

Die Aufgabe wird durch die Konstruktion eines Handgerätes gelöst.
Das Handgerät besteht aus einer fest verankerten Elektrode, die mit einer austauschbaren Biomembran bedeckt ist, einem Systemlösungs-Vorratsbeutel und einem Abfallbeutel, einer Pumpe, einem Schlauch-Transportsystem, einem Bedienhebel mit Ventilfunktion, drei Bedienelementen, einem Display und einer Steuereinheit für die Signalerfassung und den Gesamtablauf.
Das Gerät arbeitet mit einer Temperaturkompensation, so daß veränderte Empfindlichkeiten des Biosensors über Schwankungen der Umgebungstemperaturen im Bereich von 15 °C bis 35 °C auf der Basis einer Signal-Temperatur-Funktion ausgeglichen werden.
Als Vorratsbeutel für die Systemlösung sowie für den Abfall werden bevorzugt verschweißte PE-Folien verwendet, wobei das Vorratsvolumen ca 1/3 und das Abfallvolumen ca. 2/3 des Gesamtvolumens einnimmt. In einer bevorzugten Ausführungsvariante sind die beiden Beutel miteinander verschweißt, sie werden übereinander angeordnet und nach Verbrauch des Puffervorrates gemeinsam entsorgt. Die Öffnung der Beutel erfolgt beim Einsatz in das Handgerät durch eine Kanüle. Beim Neueinsatz eines Vorratsbeutels ist die zweite Hälfte des PE-Beutels leer. In dem Maße wie der Vorrat zum Reinigen der Meßzelle verbraucht wird, wird der Abfallbeutel gefüllt. Nach vollständigem Verbrauch der Systemlösung wird der Gesamtbeutel entsorgt.

Die festverankerte Elektrode ist eine Pt-Ag/AgCl-Elektrode vom Typ einer CLARK-Elektrode. Mit dem Kombinieren mit einer konfektionierten Biomembran durch einfaches Auflegen derselben wird die Elektrode zum Biosensor, der entsprechend dem verwendeten Biomolekül verschiedene Substanzen registrieren kann.
Zur Durchführung einer Messung wird der Bedienhebel aus der Ruheposition B (9) in Position A (10) gebracht und damit das Fließsystem geöffnet. Durch Drehen der Pumpe in die Stellung I wird die Elektrode mit dem Wegsaugen der davorstehenden Systemlösung in den meßbereiten Zustand versetzt.
Die Zuführung der zu analysierenden Probe erfolgt durch Einstellen der frisch genommenen Probe in eine Kapillare in die Probeöffnung des Handgerätes, die sich unmittelbar über dem Biosensor befindet. Anschließend wird die Pumpe in die Stellung II gesetzt und damit die zu messende Probe vor den Biosensor transportiert. Nach erfolgter Messung wird das Resultat im Display angezeigt und der Bediener zur Reinigung des Systems aufgefordert. Die Kapillare wird durch den Nutzer entnommen, und mit dem Zurücklegen des Bedienhebels in Position B wird das Reinigungssystem wieder geschlossen. Die Reinigung selbst wird durch langsames Drehen der Pumpe realisiert, solange bis im Display die Ausschrift ready for use erscheint, was als Bestätigung der abgeschlossenen Reinigung gilt und die Pumpe in Stellung III steht.
Als Pumpe wird bevorzugt eine Schlauchpumpe eingesetzt.
Die drei Bedienelemente sind für folgende Funktionen zuständig:
B1: Ein/Aus
B2: Menüfunktionen
B3: Set-Taste
Die Stromversorgung ist sowohl über einen internen 9 V Accumulator als auch über eine externe Solarzelle bzw. ein Netzteil möglich.
Eine Biomembran hat in Abhängigkeit vom verwendeten Biomolekül eine befristete Lebensdauer, die im allgemeinen zwischen 10 und 30 Tagen liegt. Der Ablauf der Lebensdauer wird dem Nutzer im Display angezeigt. Nach Ablauf der Lebensdauer wird die Biomembran durch eine neue ersetzt.
Der Austausch der Biomembran erfolgt durch Entriegelung der entsprechenden Verschlußkappe, der Entnahme der verbrauchten Membran und dem einfachen Auflegen der neuen Membran. Aus den im Display angezeigten Hinweisen wird der Nutzer bis zur Funktionsfähigkeit der neuen Membran über die notwendigen Arbeitsschritte informiert.
Die Signalerfassung und der Gesamtablauf wird über eine Steuereinheit geregelt. Der Gesamtablauf beinhaltet den Beginn der Signalerfassung (Start), das Ende der Signalerfassung, die Ablage und Speicherung der Meßwerte, die Eichung des Gerätes und die Display-Informationen für den Bediener bezüglich der Arbeitsschritte.

Das erfindungsgemäße Handgerät ermöglicht ein Minimum von 300 Messungen, ohne daß der Vorratsbeutel ausgewechselt werden muß. Ein besonderer Vorteil liegt darin, daß das Gerät unabhängig vom Einsatzort und der Einsatzzeit im Temperaturbereichvon 15 °C bis 35 °C einsatzfähig ist. Darüber hinaus liegt das Gewicht des Gerätes unter 500 g.

Durch die folgenden Ausführungsbeispiele und Zeichnungen wird die Erfindung näher erläutert.
Fig.1 - Gesamtansicht des Handgerätes
Fig.2 - Prinzipdarstellung des Gerätes und der Anordnung von Vorrats- und Abfallbeutel
Fig.3 - Ansicht der Elektrodenanordnung und Darstellung Biomembrantausch

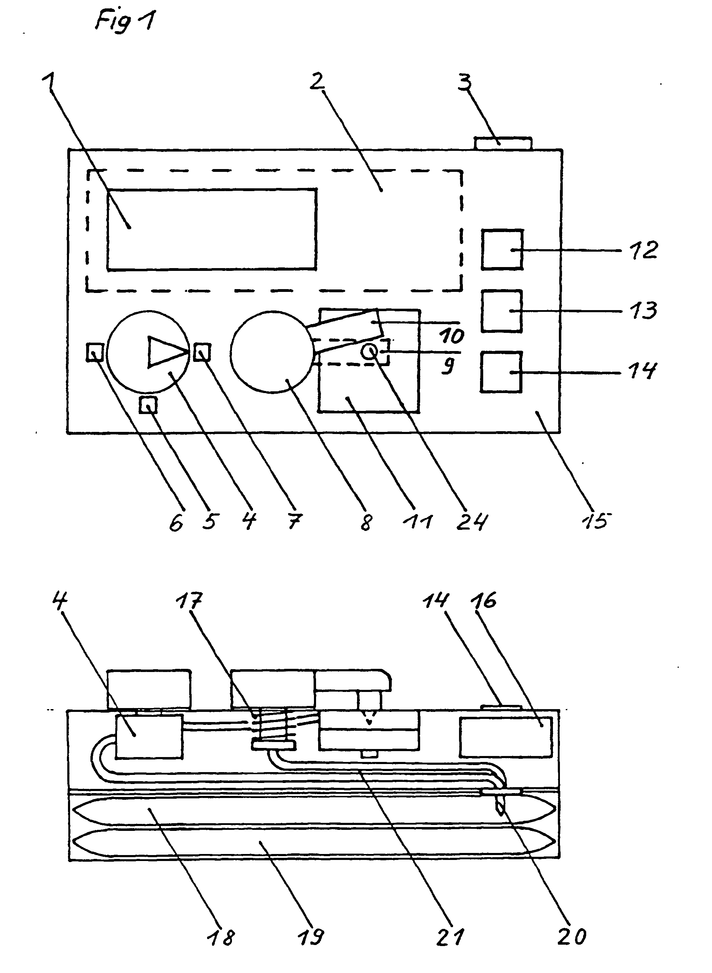

Das Handgerät gemäß Fig. 1 dient zur Untersuchung von Vollblut, Liquor, Gewebsflüssigkeit, Urin, Serum, Plasma, Lebensmittelproben sowie Wasserproben bezüglich von Metaboliten wie z.B. Glucose, Lactat; aber auch von Nährstoffen sowie Produkten der Lebensmittel- und Fermentationsindustrie wie z.B. Lactose, Ascorbinsäure, Äpfelsäure und verschiedene Aminosäuren.

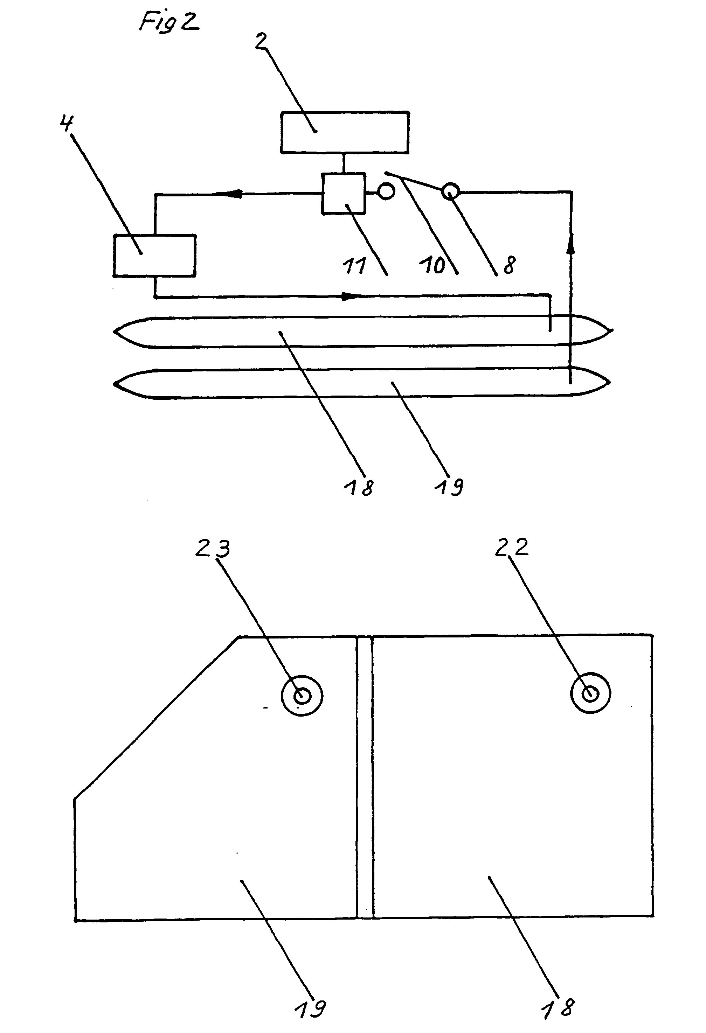

In Fig. 1,2 und 3 ist der prinzipielle Ablauf einer solchen Messung demonstriert. Bei geöffnetem Bedienhebel 8 (Position A - 10) wird das Puffersegment mit der Pumpe 4 in die Position I (5) gebracht, eine Kanüle mit zu analysierender Probe in die Öffnung 24 gestellt, die Pumpe 4 in Position II (6) gebracht, und damit im Probekanal 25 über den Biosensor 33 positioniert, womit die Messung beginnt. Nach abgeschlossener Messung wird im Display (1) der Meßwert angezeigt und der Bediener zum Reinigen des Systems aufgefordert. Dazu wird die Probekanüle aus der Öffnung 24 entnommen, der Bedienhebel 8 in Position B (9) und durch Drehen an der Pumpe 4 Systemlösung aus dem Vorratsbeutel 19 über das Schlauch-Transportsystem 21 in den Abfallbeutel bewegt. Dieser Kontakt des Biosensors mit der Pufferlösung führt zur Reinigung des Sensors. Der Bediener wird mit der Ausschrift ready for use im Display 1 über den Abschluß der Reinigung informiert. Die Pumpe wird jetzt in die Ruhestellung III (7) gebracht. Damit ist das Handgerät wieder meßbereit und die darauffolgende Messung läuft nach dem gleichen Schema ab.

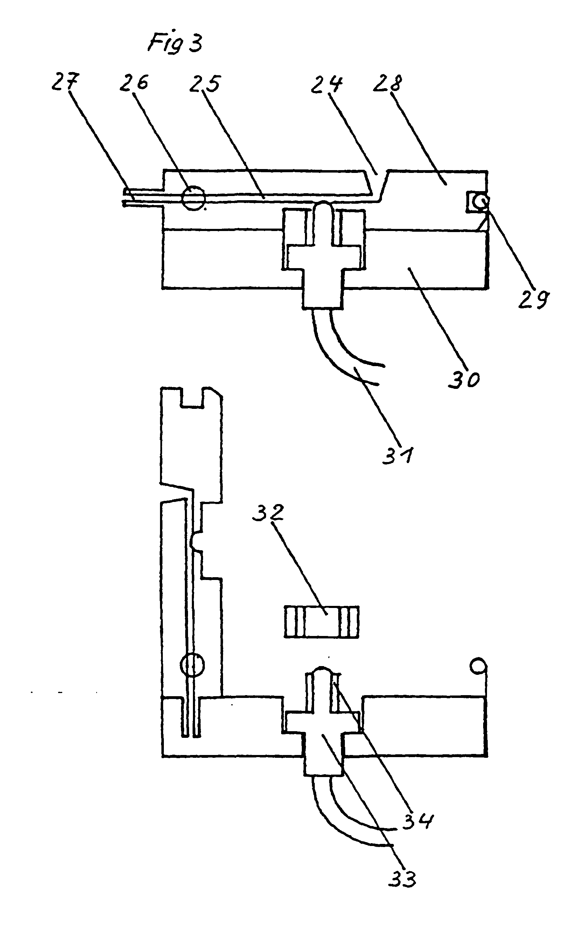

Gemäß Fig. 3 kann nach Ablauf der Lebensdauer einer Biomembran (was im Display 1 dem Bediener angezeigt wird) die Biomembran ausgetauscht werden. Dazu wird der Bedienhebel 8 über die Feder 17 nach oben gezogen und um 90° nach oben gedreht, sodaß die Meßzellenabdeckung 28 an der Achse 26 durch Lösen der Arretierung 29 um 90° nach oben geklappt werden kann. Jetzt wird die verbrauchte Biomembran 32 entnommen und eine neue auf die Elektrode 33 gelegt, die mit einer Gummidichtung 34 versehen ist. Danach wird die Meßzellenabdeckung wieder nach unten geklappt, in 29 arretiert und der Bedienhebel wieder in Position B (9) gebracht Damit ist das Fließsystem wieder geschlossen und durch das Drehen der Pumpe wird zur Konditionierung Systemlösung über den Biosensor gebracht.

### Bezugszeichen:

- 1: Display
- 2: Auswerteeinheit
- 3: Anschluß für Solarzelle bzw. Netzteil
- 4: Pumpe
- 5: Pumpe-Stellung I
- 6: Pumpe-Stellung II
- 7: Pumpe-Stellung III
- 8: Bedienhebel
- 9: Bedienhebel - Position B
- 10: Bedienhebel - Position A
- 11: Meßzelle mit Biosensor
- 12: Bedienelement B1
- 13: Bedienelement B2
- 14: Bedienelement B3
- 15: Gehäuse
- 16: Accumulator
- 17: Feder für Bedienhebel
- 18: Abfallbeutel
- 19: Vorratsbeutel
- 20: Kanüle (Verbindung Schlauchsystem - Abfall- bzw. Vorratsbeutel)
- 21: Schlauchsystem
- 22: Verschluß Abfallbeutel
- 23: Verschluß Vorratsbeutel
- 24: Probe-Öffnung
- 25: Probekanal
- 26: Achse
- 27: Anschluß zum Schlauch-Transportsystem (Verbindung Probekanal-Schlauch)
- 28: Meßzellenabdeckung
- 29: Arretierung der Meßzellenabdeckung
- 30: Meßzellenboden
- 31: Anschlüsse Elektrode
- 32: Biomembran
- 33: Elektrode
- 34: Elektrodendichtung

## Patentansprüche

1. Mobiles Handgerät mit Biosensor, insbesondere für die dezentrale Bestimmung von originalen biologischen Lösungen, **gekennzeichnet durch** einen amperometrischen Biosensor (33), bestehend aus einer Elektrode mit aufgelegter Biomembran.
eine Meßzelle (11),
Vorrats- (19) und Abfallbeutel (18) für frische und verbrauchte Systemlösung,
eine Pumpe (4),
ein Schlauch-Transportsystem (21),
eine Proben-Öffnung (24) und einen Probekanal (25),
einen Bedienungshebel mit Ventilfunktion (8),
eine Membrantauschvorrichtung (26, 27, 28, 29)
ein Display (1) zur Anzeige des Messwertes nach abgeschlossener Messung, zur Anzeige von Informationen für den Bediener bezüglich der Arbeitsschritte, zur Anzeige des Schriftzuges "ready for use" als Bestätigung der abgeschlossenen Reinigung und zur Anzeige des Ablaufs der Lebensdauer der Biomembran
ein Bedienungselement zum Ein- oder Ausschalten einer elektrischen Stromversorgung (12),
ein Bedienungselement zum Einstellen von Menüfunktionen (13),
eine Set-Taste (14),
einen 9V-Akkumulator (16),
einen Solarzellen- bzw. Netzteilanschluß (3),
eine Auswerteeinheit für die Signalerfassung und den Gesamtablauf des Meßprozesses (2) und
ein Gehäuse (15).

2. Mobiles Handgerät mit Biosensor nach Anspruch 1, **dadurch gekennzeichnet, daß** sich der Biosensor (33) unmittelbar unter der Probeöffnung (24) befindet.

3. Mobiles Handgerät mit Biosensor nach Anspruch 1 und 2, **dadurch gekennzeichnet, daß** die eingesetzte Pumpe (4) per Hand und ohne Elektroenergieeinsatz bewegt wird.

4. Mobiles Handgerät mit Biosensor nach Anspruch 1-3, **dadurch gekennzeichnet, daß** für den Vorrats- (19) und den dazugehörigen Abfallbeutel (18) verschweißte PE-Folie verwendet wird, die beiden Beutel miteinander verschweißt sind, übereinander angeordnet werden und nach Verbrauch des Puffervorrates gemeinsam entsorgt werden.

5. Mobiles Handgerät mit Biosensor nach Anspruch 1-4, **dadurch gekennzeichnet, daß** die Stromversorgung der Elektrode sowohl über einen aufladbaren Accumulator (16) als auch über eine Solarzelle oder ein Netzteil (3) realisiert werden kann.

6. Mobiles Handgerät mit Biosensor nach Anspruch 1-5, **dadurch gekennzeichnet, daß** das Gewicht des Gerätes unter 500 g ist.

## Claims

1. Mobile hand-held appliance with biosensor, in particular for decentralised determination of original biological solutions, wherein there exists an amperometric biosensor (33), comprising an electrode with an applied bio-membrane,
a gauge head (11),
stock (19) and waste bag (18) for fresh and used system solution,
a pump (4),
a hose transport system (21),
a sample opening (24) and a sample channel (25),
an operating lever with valve function (8),
a membrane replacement device (26, 27, 28, 29),
a display (1) to show the measured value after completed measurement, to show information for the user with regard to the work steps, to show the "ready for use" notification as confirmation of completion of cleaning and to show the expiry of the service life of the bio-membrane,
an operating element to switch an electric energy supply on or off (12),
an operating element to set the menu functions (13),
a set key (14),
a 9V accumulator (16),
a solar cell or power pack connection (3),
an evaluation unit for recording signals and the overall sequence of the measurement process (2) and
a housing (15).

2. Mobile hand-held appliance with biosensor according to Claim 1, wherein the biosensor (33) is positioned directly underneath the sample opening (24).

3. Mobile hand-held appliance with biosensor according to Claim 1 und 2, wherein the pump (4) used is moved by hand and without the use of electrical energy.

4. Mobile hand-held appliance with biosensor according to Claim 1-3, wherein welded PE foil is used for the stock (19) and the matching waste bags (18), the two bags are welded to one another, arranged one on top of the other and disposed of jointly after consumption of the buffer stock.

5. Mobile hand-held appliance with biosensor according to Claim 1-4, wherein the energy supply of the electrode can be implemented both via an accumulator (16) and also via a solar cell or a power-pack (3).

6. Mobile hand-held appliance with biosensor according to Claim 1-5, wherein the weight of the appliance is less than 500 g.

## Revendications

1. Un appareil manuel mobile avec biocapteur, en particulier pour la détermination décentralisée de solutions biologiques originales, **caractérisées par** un biocapteur ampèremètre (33), composé d'une électrode avec une biomembrane superposée,
une cellule détectrice (11),
un sachet de réserve (19) et de déchets (18) pour la solution système fraîche et usée,
une pompe (4),
un système de transport à tuyau (21),
une ouverture à échantillon (24) et un canal à échantillon (25),
un levier de manipulation à fonction de soupape (8),
un dispositif de remplacement de la membrane (26, 27, 28, 29),
une visualisation (1) pour afficher la valeur de mesure après la mesure, pour afficher des informations pour l'opérateur en ce qui concerne les étapes opératoires, pour afficher le logo « ready for use » à titre de confirmation de l'achèvement du nettoyage et pour afficher l'expiration de la durée de vie de la biomembrane,
un élément de commande pour brancher ou débrancher une alimentation en courant électrique (12),
un élément de commande pour régler les fonctions du menu (13),
une touche de positionnement (14),
un accumulateur 9V (16),
un raccord pour cellule solaire voire bloc d'alimentation (3),
une unité d'évaluation pour la saisie du signal et le déroulement complet du processus de mesure (2) et
un boîtier (15).

2. Appareil manuel mobile avec biocapteur selon la revendication 1, **se caractérisant par le fait que** le biocapteur (33) se trouve directement sous l'ouverture à échantillon (24).

3. Appareil manuel mobile avec biocapteur selon la revendication 1 et 2, **se caractérisant par le fait que** la pompe employée (4) est déplacée à la main et sans utiliser d'énergie électrique.

4. Appareil manuel mobile avec biocapteur selon la revendication 1-3, **se caractérisant par le fait qu'**un film PE soudé est utilisé pour le sachet de réserve (19) et le sachet de déchets (18) correspondants, les deux sachets étant soudés l'un à l'autre, placés l'un au-dessus de l'autre et étant éliminés ensemble après épuisement de la réserve en tampon.

5. Appareil manuel mobile avec biocapteur selon la revendication 1-4, **se caractérisant par le fait que** l'alimentation en courant de l'électrode pourra se faire par l'intermédiaire d'un accumulateur chargeable (16) ainsi que par l'intermédiaire d'une cellule solaire voire d'un bloc d'alimentation (3).

6. Appareil manuel mobile avec biocapteur selon la revendication 1-5, **se caractérisant par le fait que** le poids de cet appareil est inférieur à 500 g.
